# EUROPEAN PATENT SPECIFICATION

(11) **EP 4 028 559 B1**
(45) Date of publication and mention of the grant of the patent: **07.01.2026**
(21) Application number: 20851950.4
(22) Date of filing: 15.08.2020
(51) Int. Cl.: C12Q 1/70, C12Q 1/6886

(54) **METHODS FOR DIAGNOSIS AND IN VITRO RISK STRATIFICATION FOR HEAD AND NECK CANCER BASED ON EXOSOMAL MRNAS**
VERFAHREN ZUR DIAGNOSE UND IN-VITRO-RISIKOSTRATIFIZIERUNG FÜR KOPF-HALS-KARZINOM AUF DER GRUNDLAGE VON EXOSOMALEN MRNA
PROCÉDÉS DE DIAGNOSTIC ET DE STRATIFICATION DES RISQUES IN VITRO POUR LE CANCER DE LA TÊTE ET DU COU SUR LA BASE DE MARN EXOSOMAUX

(30) Priority: 15.08.2019 IN 201931019425
(43) Date of publication of application: 20.07.2022
(73) Proprietor: Shrestha, Devjani Ghosh, West Bengal 700106 (IN)
(72) Inventor: Shrestha, Devjani Ghosh, West Bengal 700106 (IN)
(74) Representative: Cleveland Scott York
(86) International application number: PCT/IN2020/050715
(87) International publication number: WO 2021/028950

(56) References cited:
- WO-A1-2010/129941
- WO-A1-2019/152059
- WO-A2-03/019143
- WO-A2-2016/005905
- CN-A- 110 982 814
- CN-B- 101 709 335
- US-A1- 2010 120 019
- US-A1- 2011 229 876
- US-A1- 2014 213 472
- DATABASE Geneseq [online] 11 June 2020 (2020-06-11), "HPV 16 target gene detecting probe HPV16-Probe, SEQ ID 27.", retrieved from EBI accession no. GSN:BHR86605 Database accession no. BHR86605
- DATABASE Geneseq [online] 10 May 2003 (2003-05-10), "Fluorescent multiplex HPV PCR assay primer #15.", retrieved from EBI accession no. GSN:ABX12345 Database accession no. ABX12345
- DATABASE Geneseq [online] 30 September 2010 (2010-09-30), "HPV16 E6 protein gene downstream PCR primer HPV16R.", retrieved from EBI accession no. GSN:AYG56078 Database accession no. AYG56078
- DATABASE Geneseq [online] 8 July 2010 (2010-07-08), "HPV16 E7 transcripts specific PCR antisense primer sequence.", retrieved from EBI accession no. GSN:AYA71840 Database accession no. AYA71840
- WANG ZEYU ET AL: "Acoustofluidic Salivary Exosome Isolation", THE JOURNAL OF MOLECULAR DIAGNOSTICS, vol. 22, no. 1, 1 January 2020 (2020-01-01), pages 50 - 59, XP093072180, ISSN: 1525-1578, DOI: 10.1016/j.jmoldx.2019.08.004
- KANNAN ANBARASU ET AL: "Genetic Mutation and Exosome Signature of Human Papilloma Virus Associated Oropharyngeal Cancer", SCIENTIFIC REPORTS, vol. 7, no. 1, 6 April 2017 (2017-04-06), XP093072232, DOI: 10.1038/srep46102
- LUDWIG SONJA ET AL: "Molecular and Functional Profiles of Exosomes From HPV(+) and HPV(-) Head and Neck Cancer Cell Lines", FRONTIERS IN ONCOLOGY, vol. 8, 12 October 2018 (2018-10-12), XP093072168, DOI: 10.3389/fonc.2018.00445
- DATABASE Nucleotide 14 March 2019 (2019-03-14), "Human papillomavirus type 16 K1753 DNA, complete genome Sequence ID", XP055793128, retrieved from NCBI Database accession no. LC456606.1
- SALAZAR, CAROLINA ET AL.: "A novel saliva-based microRNA biomarker panel to detect head and neck cancers", CELLULAR ONCOLOGY, vol. 37, no. 5, 2014, pages 331 - 338, XP035400186, DOI: 10.1007/s13402-014-0188-2
- ANDERSEN ANNE SKOU, KOLDJAER SØLLING ANNE SOPHIE, OVESEN THERESE, RUSAN MARIA: "The interplay between HPV and host immunity in head and neck squamous cell carcinoma", INTERNATIONAL JOURNAL OF CANCER, vol. 134, no. 12, 2014, pages 2755 - 2763, XP055793131
- LIANG CAIHUA, MARSIT CARMEN J., MCCLEAN MICHAEL D., NELSON HEATHER H., CHRISTENSEN BROCK C., HADDAD ROBERT I., CLARK JOHN R., WEIN: "Biomarkers of HPV in head and neck squamous cell carcinoma", CANCER RESEARCH, vol. 72, no. 19, 2012, pages 5004 - 5013, XP055793139
- DATABASE Nucleotide NCBI; 18 November 2020 (2020-11-18), "Human papillomavirus type 16 isolate VRDL/HPV-467/2019/AIIMS Raipur/Ind-CG E6 gene, complete cds GenBank", XP055793152, retrieved from NCBI Database accession no. MN987228.1

## Description

The following specification particularly describes the invention and the manner in which it is to be performed.

### CROSS REFERENCE TO RELATED APPLICATION

The present application claims priority from Indian Provisional Patent Application No. 201931019425

### FIELD OF THE INVENTION

The present invention pertains to diagnostic and in vitro risk stratification methods. More particularly, the invention relates to diagnostic and in vitro risk stratification methods for Human Papilloma Virus positive head and neck cancer based on exosomal mRNA expression.

### BACKGROUND OF THE INVENTION

Head and neck squamous cell carcinoma (HNSCC) represents one of the leading causes of cancer deaths across the world. HNSCC is the 6th most common cancer worldwide and 5th most common cancer in the South East Asia region. The incidence varies widely around the world and within populations. Oral and oropharyngeal cancer constitutes 3-5% of the malignancies in Europe, while in parts of SE Asia and India, it reaches up to 40-50%. About, 40,000 pharyngeal cancers occur in India every year.

About 70% of cancers in the oropharynx (which includes the tonsils, soft palate, and base of the tongue) are linked to human papillomavirus (HPV). In particular, HPV-16 is recognized to be responsible, with different epidemiological, clinical, anatomical, radiological, behavioral, biological and prognostic characteristics from HPV negative oropharyngeal squamous cell carcinoma, a type of head and neck squamous cell carcinoma.

It is a recognized fact that the survival chance of a person becomes better if head and neck cancer is diagnosed and treated at earlier stages. However, due to limited resources, poverty, inadequate manpower, poor health literacy, and social taboos surrounding cancer in developing and less developed countries, most of the cancer incidences are detected at advanced stages. Most of the cancer patients in India are diagnosed at stages III and IV.

The aforementioned scenario coupled with factors such as low investment in healthcare and a low ratio of oncologists to cancer cases enhances the cancer mortality rates. Therefore, it becomes extremely important to screen head and neck cancer at an early stage to prevent cancer-related deaths.

Currently, HPV tests that have been approved by the United States Food and Drug Administration (FDA) are those intended for use in cervical carcinoma. There are no HPV diagnostic tests with regulatory approval for oropharyngeal cancer. Rather, the HPV testing options available for use in the clinical setting for head and neck cancer are laboratory-developed tests (LDTs), which can involve various techniques and platforms for the analysis of HPV DNA, HPV messenger RNA (mRNA). or the p16 protein.

The methods currently used for diagnosis of HPV positive head and neck cancer includes biopsy to extract sample cells or tissues, followed by p16 detection by Immunohistochemistry, which is a surrogate marker and can provide prognostic information.

HPV DNA/RNA detection by PCR or In Situ Hybridization is done entails extremely high cost and expertise. Therefore, these tests are not available for most patients across the world.

WANG ZEYU ET AL: "Acoustofluidic Salivary Exosome Isolation", THE JOURNAL OF MOLECULAR DIAGNOSTICS, vol. 22, no. 1, 1 January 2020, pages 50-59 (ISSN: 1525-1578, DOI: 10.1016/j.jmoldx.2019.08.004) discloses a method for detection of HPV16 viral DNA in exosomes obtained from saliva of HPV-associated oropharyngeal cancer patients. The method involves exosome purification and amplification / detection of HPV16 DNA by droplet digital PCR.

However, the currently used methods suffer from one or more of the following limitations:
- The methods are invasive, which is painful for the patient.
- The methods are not cost-effective.
- The methods are time-consuming.
- The methods are not commercially available in most parts of the world.
- The majority of the methods are not validated for the diagnosis of head and neck cancers.
- The methods require trained personnel for carrying out the biopsy.
- The available methods do not detect clinically significant HPV infection.
- The expression levels of E6/E7 genes varies from sample to sample, and it is not possible to conclusively determine the extent and spread of cancer.
- Real-time therapy and disease monitoring are problematic due to the absence of visible tumor tissue following successful treatment. It is difficult to conduct biopsy repeatedly in case of suspected recurrence.
- Treatment outcomes cannot be immediately assessed, which may result in late therapeutic intervention leading to recurrence of the disease.

Therefore, there is a need for diagnostic kits and methods for detection which can successfully overcome the limitations of the prior art. The present invention overcomes the problems of the prior art by solving this long-standing problem for rapid, non-invasive, and inexpensive determination of head and neck cancers. The invention would enable head and neck cancer screening more accessible and affordable to a huge proportion of the population living around the world.

### SUMMARY OF THE INVENTION

### Technical Problem

The technical problem to be solved in this invention is the rapid, non-invasive, and inexpensive diagnosis of HPV positive head and neck squamous cell carcinoma.

### The solution to the problem

The problem has been solved by developing a set of primers that can be used for diagnosis of HPV positive head and neck squamous cell carcinoma.

### Overview of the invention

The present disclosure provides for primers, kits, PCR assay methods, and methods for in vitro and non-invasive diagnosis of HPV positive head and neck squamous cell carcinoma. The invention provides inexpensive, rapid, non-invasive, and objective determination whether a person is affected with HPV positive head and neck squamous cell carcinoma. Non-requirement of trained personnel for sample collection provides a huge advantage and can lead to real-time disease monitoring.

### BRIEF DESCRIPTION OF THE DRAWINGS

Figure 1: Figure 1A provides a non-limiting embodiment for the collection of exosomes from salivary samples. Figure 1B provides a non-limiting embodiment for RNA isolation from exosomes.
Figure 2: Representative images of semi-quantitative PCR for E6 (108bp) and E7 (100bp) primers in serum. A) represents Serum E6 gene expression in HNCC patient samples and SiHa, Caski cells. B) represents Serum E7 gene expression in HNCC patient samples and SiHa, Caski cells.
Figure 3: Representative images of semi-quantitative PCR for E6(108bp) and E7(100bp) primers in saliva. A) represents Saliva E6 gene expression in HNCC patient samples and SiHa, Caski cells. B) represents Saliva E7 gene expression in HNCC patient samples and SiHa, Caski cells.
Figure 4: Representative images of semi-quantitative PCR for E6(108bp) and E7(100bp) primers in Tissue. A) represents Tissue E6 gene expression in HNCC patient samples and SiHa, Caski cells. B) represents Tissue E7 gene expression in HNCC patient samples and SiHa, Caski cells.
Figure 5: Expression of E6 and E7 genes during semi-quantitative PCR
Figure 6: Results of a semi-quantitative PCR in which the expression level of E6 and E7 genes of HPV16 in salivary exosomes and tumor tissues were compared with the expression levels of GAPDH.
Figure 7: Representative images of semi-quantitative PCR for E6(108bp) and E7 (100bp) primers in serum. A) represents Serum E6 gene expression in HNCC patient samples and SiHa, Caski cells. B) represents Serum E7 gene expression in HNCC patient samples and SiHa, Caski cells.
Figure 8: Representative images of semi-quantitative PCR for E6(108bp) and E7(100bp) primers in saliva. A) represents Saliva E6 gene expression in HNCC patient samples and SiHa, Caski cells. B) represents Saliva E7 gene expression in HNCC patient samples and SiHa, Caski cells.
Figure 9: Representative images of semi-quantitative PCR for E6(108bp) and E7(100bp) primers in Tissue. A) represents Tissue E6 gene expression in HNCC patient samples and SiHa, Caski cells. B) represents Tissue E7 gene expression in HNCC patient samples and SiHa, Caski cells.
Figure 10: Expression of E6 and E7 genes during quantitative PCR.

### BRIEF DESCRIPTION OF SEQUENCE LISTING

SEQ ID NO: 1 is the coding region sequence on E6 gene which can be amplified by SEQ ID NO: 3 and SEQ ID NO: 4.
SEQ ID NO: 2 is the coding region sequence on E7 gene which can be amplified by SEQ ID NO: 5 and SEQ ID NO: 6.
SEQ ID NO: 3 is the forward primer of the primer set 1 (E6 primer) as used herein.
SEQ ID NO: 4 is the reverse primer of the primer set 1 (E6 primer) as used herein.
SEQ ID NO: 5 is the forward primer of the primer set 2 (E7 primer) as used herein.
SEQ ID NO: 6 is the reverse primer of the primer set 2 (E7 primer) as used herein.
SEQ ID NO: 7 and SEQ ID NO: 8 represents GAPDH forward and reverse primers.

### DEFINITION

Unless defined otherwise, all technical and scientific terms used herein have the same meaning as commonly understood by one of ordinary skill in the art to which the methods belong. Although any method, device, kit, reagent, or a composition similar or equivalent to those described herein can also be used in the practice or testing of the methods, representative illustrative methods and compositions are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements or use of a "negative" limitation.

In the present invention, the term "exosomes" refers to small vesicles having a membrane structure secreted from various cells. Exosomes have diameters of about 30 to 100 nm, and fusion between plasma membranes and multivesicular bodies occurs, thereby being released to the outside of the cells. As used herein, "exosomal DNA" refers to DNA isolated from exosomes of a biological sample. As used herein, "exosomal mRNA" refers to mRNA isolated from exosomes of a biological sample.

The term "exosomal sample" may encompass both exosomal DNA and exosomal mRNA. Further, the term "exosomal sample" indicates, exosomes collected from same/different biological samples by non-invasive means. In a non-limiting embodiment, the term encompasses exosomes collected from saliva, exosomes collected from serum, a combination of exosomes collected from saliva and serum, and the like.

As used herein, the term "about" refers to an understood variation from the stated value. It is to be understood that such a variation is always included in any given value provided herein, whether or not it is specifically referred to.

The term "primer" refers to a nucleic acid capable of acting as a point of initiation of template-directed nucleic acid synthesis when placed under conditions in which polynucleotide extension is initiated (e.g., under conditions comprising the presence of requisite nucleoside triphosphates (as dictated by the template that is copied) and a polymerase in an appropriate buffer and at a suitable temperature or cycle(s) of temperatures (e.g., as in a polymerase chain reaction)). A primer is typically a single-stranded oligonucleotide, typically having a range from 6 to 40 nucleotides. As used herein, the term is used to refer to an oligonucleotide selected from a group comprising SEQ ID NO: 3, SEQ ID NO: 4, SEQ ID NO: 5 and SEQ ID NO: 6.

The term "forward primer" means a primer that anneals to the anti-sense strand of dsDNA. As used herein, the term is used to refer to an oligonucleotide selected from a group comprising SEQ ID NO: 3 and SEQ ID NO: 5.

The term "reverse primer" means a primer that anneals to the sense strand of dsDNA. As used herein, the term is used to refer to an oligonucleotide selected from a group comprising SEQ ID NO: 4 and SEQ ID NO: 6.

The phrase "set of primers" as used herein, refers to a composition comprising two forward primers (SEQ ID NO: 3 and SEQ ID NO: 5) and two reverse primers (SEQ ID NO: 4 and SEQ ID NO: 6) that can be used together to amplify a given region of a nucleic acid-specific to E6 or E7 gene of HPV16.

As defined herein, "biological sample" refers to a body fluid containing cells from which exosomal mRNA can be obtained by non-invasive means. For example, the biological sample can be derived from blood, saliva, cerebral spinal fluid, sputa, urine, mucous, synovial fluid, peritoneal fluid, amniotic fluid, and the like. The biological sample can be used either directly as obtained from the source or following a pretreatment to modify the character of the sample. Thus, the biological sample can be pre-treated before use by, for example, preparing plasma or serum from blood, disrupting cells, preparing liquids from solid materials, diluting viscous fluids, filtering liquids, distilling liquids, concentrating liquids, inactivating interfering components, adding reagents, and the like.

As used herein, the term "subject" encompasses any human being. In a non-limiting embodiment, the human subject is already diagnosed or is exhibiting symptoms indicative of head and neck squamous cell carcinoma.

The term "sensitivity" as used herein refers to the probability that a test result will be positive when the disease is present (true positive rate).

The term "specificity" as used herein refers to the probability that a test result will be negative when the disease is not present (true negative rate).

The term "positive likelihood ratio" as used herein refers to the ratio between the probability of a positive test result given the presence of the disease and the probability of a positive test result given the absence of the disease, i.e. Positive likelihood ratio = True positive rate / False positive rate = Sensitivity / (1-Specificity).

The term "negative likelihood ratio" as used herein refers to the ratio between the probability of a negative test result given the presence of the disease and the probability of a negative test result given the absence of the disease, i.e. Negative likelihood ratio = False-negative rate / True negative rate = (1-Sensitivity) / Specificity.

The term "accuracy" as used herein refers to the overall probability that a patient is correctly classified. Accuracy = Sensitivity × Prevalence + Specificity × (1 - Prevalence)

### DETAILED DESCRIPTION OF THE INVENTION.

The present invention is defined by the apended claims.

The present specification discloses methods for predicting, diagnosing, and monitoring head and neck cancer.

For the first time, the inventors have developed a method for in vitro diagnosis and risk stratification based on expression levels of E6 and E7 genes of HPV16 in exosomes isolated from saliva and serum.

The present invention represents an advancement over the existing methods for the determination of HPV positive head and neck cancer. The advances are characterized by the following features:
(a) Affordable: The diagnostic and risk stratification methods developed in the present invention are inexpensive as compared to existing methods as they are non-invasive and do not require the involvement of trained personnel.
(b) Sensitive and Specific: The measurement of E6 and E7 mRNA levels of enables accurate and objective determination of regarding the presence/absence of clinically significant HPV positive head and neck cancer.
(c) Rapid: Being non-invasive, test results can be ascertained within a short period.
(d) Assessing response and recurrences: The methods of the present invention would enable assessment of response to treatment and detect early recurrence in cases of HPV positive head and neck cancers

The technical problem to be solved in this invention is rapid, inexpensive and non-invasive prediction, diagnosis, and monitoring of HPV positive head and neck cancer.

The inventors have solved the above problem by the development of a primer set which can determine whether a person is affected by HPV-positive head and neck cancer based on the expression levels of E6 and E7 genes of HPV16 in exosomes isolated from saliva and serum.

The inventors have contemplated a unique approach by analyzing E6 and E7 genes of HPV16 expressed in exosomes. Based on the analysis, the inventors have designed two sets of primers, which can be utilized for identifying E6 and E7 genes of HPV16 expressed in exosomes isolated by non-invasive means. The primer sets are highly effective and reduce the time and efforts taken for the identification of head and neck squamous cell carcinoma.

Before the methods of the present disclosure are described in greater detail, it is to be understood that the invention is not limited to particular embodiments and may vary. It is also to be understood that the terminology used herein is for the purpose of describing particular embodiments only and is not intended to be limiting.

The present invention allows the identification and characterization of targets useful for prognosis, diagnosis and monitoring, and/or other therapeutic intervention of cancers.

### Sequence analysis of E6 and E7 genes of HPV16

In HPV16, E6 and E7 proteins are known to play an important role in head and neck carcinogenesis. The E6 and E7 genes from Papillomavirus Episteme (PaVE) genome database maintained by NIAID, NIH (https://pave.niaid.nih.gov/) were studied and two target sequences (coding regions) in the E6 and E7 genes were identified for the development of the primers.

The Human papillomavirus type 16 isolate HP_2013-128 E6 protein (E6) and E7 protein (E7) genes (complete CDS) is available under GenBank Accession Number: MH370229.1, and originally published as Pham, Trang Thi Thu, et al. "Human papillomavirus genotypes and HPV16 E6/E7 variants among patients with genital cancers in Vietnam." *Japanese journal of infectious diseases* (2018): JJID-2018.
E6 coding region sequence (SEQ ID NO: 1) 
atgtttcaggacccacaggagcgacccagaaagttaccacagttatgcacagagctgcaaacaactatacatgatataatattagaatgt
gtgtactgcaagcaacagttactgcgacgtgaggtatatgactttgcttttcgggatttatgcatagtatatagagatgggaatccatatgc
tgtatgtgataaatgtttaaagttttattctaaaattagtgagtatagacattattgttatagtttgtatggaacaacattagaacagcaatacaa
caaaccgttgtgtgatttgttaattaggtgtattaactgtcaaaagccactgtgtcctgaagaaaagcaaagacatctggacaaaaagca
aagattccataatataaggggtcggtggaccggtcgatgtatgtcttgttgcagatcatcaagaacacgtagagaaacccagctgtaa
E7 Coding region sequence (SEQ ID NO: 2)

atgcatggagatacacctacattgcatgaatatatgttagatttgcaaccagagacaactgatctctactgttatgagcaattaaatgacag
ctcagaggaggaggatgaaatagatggtccagctggacaagcagaaccggacagagcccattacaatattgtaaccttttgttgcaag
tgtgactctacgctcggttgtgcgtacaaagcacacacgtagacattcgtactttggaagacctgttaatgggcacactaggaattgtgtg
ccccatctgttctcagaaaccataa

Based on the above identified coding region, two primer sets were developed for identification of E6 and E7 genes of HPV16 in exosomal mRNA isolated from saliva and serum.

### Development of primers

Based on the sequence analysis, the primers were designed. For designing the primer, an open-source online primer design software, NCBI primer blast software plus was used. Any designing software known to a skilled person can be used. Preferably, the software can be programmed to avoid issues arising out of sequence quality such as avoidance of repetitive elements.

In one embodiment, two primer sets were created, and the molecular markers are selected from the primer set 1 (E6 primers) and primer set 2 (E7 primers).

### E6 primer set

In one embodiment, the forward primer of the E6 primer set comprises the nucleotide sequence of SEQ ID NO:3, which is GCGACCCAGAAAGTTACCACA.

In another embodiment, the reverse primer of the primer set 1 (E6 primer) comprises the nucleotide sequence of SEQ ID NO: 4, which is AAGTCATATACCTCACGTCGCA.

The primer set 1 can amplify E6 coding region represented by SEQ ID NO: 1 of E6 gene of HPV16 in exosomal mRNA isolated from saliva and serum.

The length of the product as a result of amplification by E6 primer set is 114 bp.

### E7 primers

In yet another embodiment, the forward primer of the primer set 2 (E7 primer) comprises the nucleotide sequence of SEQ ID NO: 5, which is CAGAACCGGACAGAGCCCATT.

In yet another embodiment, the reverse primer of the primer set 2 (E7 primer) comprises the nucleotide sequence of SEQ ID NO: 6, which is AACAGATGGGGCACACAATTCC.

The primer set 2 can amplify E7 coding region represented by SEQ ID NO: 2 of E7 gene of HPV16 in exosomal mRNA isolated from saliva and serum.

The length of the product as a result of amplification by E7 primer set is 150 bp.

Primers to be used in the present invention can be easily synthesized using a known method.

Preferably, the primers can be artificially synthesized using gene synthesis approach, a commonly used technique in synthetic biology that is used to create artificial nucleic acids. The state of the art makes it possible to prepare a completely synthetic double-stranded DNA molecule 'de novo' without the need for precursor template DNA. The nucleic acids synthesized through artificial gene synthesis approach is faster, economical, and less error-prone.

### Collection of biological samples from a subject

The biological samples collected for the purposes of this invention are collected through non-invasive means. The biological samples used in this invention are different from traditionally used samples, such as surgical or biopsy samples.

In one embodiment, a biological sample is collected from a head and neck tissue sample.

In another embodiment, the biological sample is collected in a non-invasive manner.

In another embodiment, the biological sample is a salivary sample.

In yet another embodiment, the biological sample is a serum sample.

In another embodiment, the biological sample can be harvested using standard techniques known in the art.

### Isolation of exosomes

In a further embodiment, the exosomes are isolated from the collected biological samples.

In another embodiment, exosomes may be isolated from the cell culture supernatant of cell lines derived from patients infected with head and neck cancer. Such cell lines may be expanded from individual cells isolated from patients infected with head and neck cancer and propagated in vitro.

In an alternative embodiment, exosomes may be isolated from the serum or saliva of an individual infected with head and neck cancer. Techniques for the separation of exosomes from serum or saliva are known to a person skilled in the art.

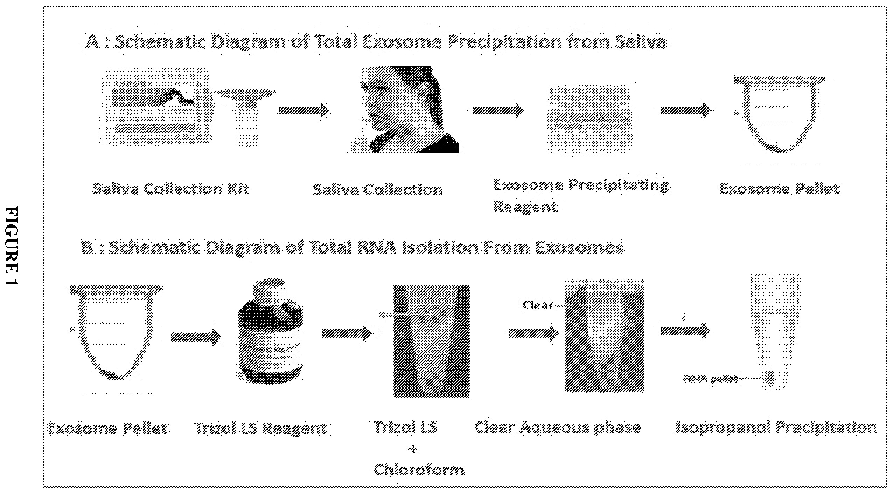

Figure 1A provides a non-limiting embodiment for the collection of exosomes from salivary samples. In the embodiment, a saliva collection kit is used for collecting the saliva. Thereafter, exosome precipitating reagent known in the art is used for precipitating the exosome in a pellet form.

In another embodiment, the exosomes can be isolated from any biological sample using standard techniques known in the art.

Exosome particles may be prepared from biological samples using any suitable technique, as will be clear to those of skill in the art.

### Checking the quality of the isolated exosomes

In another embodiment, one or more biochemical assays are performed to check the quality of the isolated exosomes.

In another embodiment, the total protein content of the isolated exosomes is checked to assess the quality of the exosomes. Total protein content may be checked using any suitable technique, as will be clear to those of skill in the art.

In another embodiment, the lipid content of the isolated exosomes is measured to assess the quality of the exosomes. Lipid content may be checked using any suitable technique, as will be clear to those of skill in the art.

In another embodiment, an acetylcholinesterase assay is performed on the isolated exosomes to assess the quality of the exosomes. Acetylcholinesterase assay may be performed using any suitable technique, as will be clear to those of skill in the art.

### PCR based assay method for measuring the expression levels of E6 and E7 genes of HPV16 in isolated exosomes

Measuring the expression level of E6 and E7 genes of HPV16 is performed using the method of the invention.

According to one embodiment of the invention, methods for measuring the levels of mRNA are provided.

In another embodiment, mRNA is isolated from the exosomes of the biological samples.

Figure 1B provides a non-limiting embodiment for RNA isolation from exosomes. In the embodiment, RNA is isolated using Trizol^{™} reagent (Invitrogen^{™}) following the manufacturer's recommended protocols.

The expression level is determined at the nucleic acid level. Typically, the expression level of a gene may be determined by determining the quantity of mRNA. Methods for determining the quantity of mRNA are well known in the art.

The PCR based assay for detecting the presence of E6 or E7 gene of HPV16 in an exosomal sample comprises the steps of:
a. isolating mRNA from exosomal samples;
b. subjecting the isolated mRNA to amplification by said PCR using primers selected from a group comprising SEQ ID NO: 3, SEQ ID NO: 4, SEQ ID NO: 5 and SEQ ID NO: 6, wherein said primer sequences are specific to bind to HPV16 gene or fragment thereof; and
c. subjecting the amplified product resulting from step (b) to electrophoresis and visualizing the fluorescent bands under UV light, for detecting the presence of said E6 or E7 gene of HPV in the said biological sample.

The primer set used is adapted for amplifying SEQ ID NO: 1 and SEQ ID NO: 2.

For example, the mRNA contained in biological samples such as exosomes is first extracted according to standard methods. The extracted mRNA is then detected by hybridization (e. g., Northern blot analysis, in situ hybridization) and/or amplification (e.g., RT-PCR).

In some embodiments, the methods of the invention comprise the steps of providing total RNAs extracted from and subjecting the RNAs to amplification and hybridization to specific probes, more particularly by means of a quantitative or semi-quantitative RT-PCR.

In a non-limiting embodiment, the steps of semi-quantitative PCR for amplifying the coding regions of E6 or E7 gene of HPV16 comprises:
a. initial denaturation at 95°C for 5 minutes;
b. thirty-five annealing cycles, each annealing cycle comprising:
   i. denaturation at 95°C for 30 seconds;
   ii. annealing at 60°C for 1 minute;
   iii. extension at 72°C for 1 minute; and
c. a final extension at 72°C for 5 min.

In another non-limiting embodiment, the steps of semi-quantitative PCR for amplifying the coding regions of E6 or E7 gene of HPV16 comprises:
a. initial denaturation at 95°C for 30 seconds;
b. forty annealing and extension cycles, each cycle comprising:
   i. denaturation at 95°C for 15 seconds; and
   ii. annealing at 60°C for 1 minute.

### Kits

The present disclosure provides diagnostic/screening kits for use in a clinical environment. Such kits could not only include one or more sampling means but other materials necessary for the identification of E6 and E7 genes of HPV16 in exosomal samples.

The kits can optionally include reagents required to conduct a diagnostic assay, such as buffers, salts, detection reagents, and the like. Other components, such as buffers and solutions for the isolation and/or treatment of a biological sample, may also be included in the kit. One or more of the components of the kit may be lyophilized and the kit may further comprise reagents suitable for the reconstitution of the lyophilized components.

Where appropriate, the kit may also contain reaction vessels, mixing vessels, and other components that facilitate the preparation of the test sample. The kit may also optionally include instructions for use, which may be provided in paper form or in computer-readable forms, such as a disc, CD, DVD, or the like.

A kit for diagnosing Head and neck squamous cell carcinoma comprising means for extraction of exosomal mRNA, primers, reagents and instructions is disclosed.

The kit comprises at least one primer set for amplifying a target sequence of HPV16 exosomal mRNA in a biological sample, wherein the target sequence has a nucleic acid sequence of SEQ ID NO: 1 or SEQ ID NO: 2.

The kit may comprise the following primer set:
d. a forward primer comprising the nucleic acid sequence of SEQ ID NO: 3;
e. a reverse primer comprising the nucleic acid sequence of SEQ ID NO: 4;
f. a forward primer comprising the nucleic acid sequence of SEQ ID NO: 5;
g. a reverse primer comprising the nucleic acid sequence of SEQ ID NO: 6.

The kit may comprise comprises a primer set comprising:
iii. a forward primer comprising the nucleic acid sequence of SEQ ID NO: 3,
iv. a reverse primer comprising the nucleic acid sequence of SEQ ID NO: 4, wherein the primer set is adapted for amplifying a target sequence of E6 gene comprising the nucleic acid sequence of SEQ ID NO: 1.

The kit may comprise a primer set comprising:
i. a forward primer comprising the nucleic acid sequence of SEQ ID NO: 5,
ii. a reverse primer comprising the nucleic acid sequence of SEQ ID NO: 6,
wherein the primer set is adapted for amplifying a target sequence of E6 gene comprising the nucleic acid sequence of SEQ ID NO: 2.

The kit may further comprise means to isolate mRNA of HPV16, means to carry out amplification of the isolated mRNA, and means to carry out electrophoresing of amplified product and to visualize fluorescent bands under UV light.

In another aspect of the disclosure there is provided a kit for diagnosing Head and neck squamous cell carcinoma, comprising means to isolate mRNA of HPV16, means to carry out amplification of the isolated mRNA, and means to carry out electrophoresing of amplified product and to visualize fluorescent bands under UV light, primers having the nucleic acid sequences recited in SEQ ID NOs: 3, 4, 5 and 6, reagents and instructions.

### In vitro methods for diagnosis of HPV positive head and neck cancer

The present invention is based on the factor that expression levels of E6 and E7 genes of HPV16 in exosomes are significantly more from the expression levels of E6 and E7 genes in other biological samples.

Diagnosis of any clinical condition contains the following phases: (i) the examination phase, involving the collection of data; (ii) the comparison of these data with standard values (iii) the finding of any significant deviation, i.e. a symptom, during the comparison; and (iv) the attribution of the deviation to a particular clinical picture.

Measurement of the mRNA expression levels of E6 and E7 genes from the biological samples such as exosomes provides for a sound basis to find any significant deviation from the standard values. Consequently, it can be determined whether the deviation is due to the presence of head and neck cancerous cells in the subject.

In one embodiment, the invention provides an in vitro non-invasive method of diagnosing head and neck squamous cell carcinoma in a subject, comprising:
a. isolating mRNA from exosomal samples of the subject;
b. amplifying, with a pair of PCR primers a target sequence of HPV16 mRNA in the exosomal sample, wherein the target sequence has a nucleic acid sequence of SEQ ID NO: 1 or SEQ ID NO: 2;
c. subjecting the amplified product resulting from step (b) to electrophoresis and visualizing fluorescent bands under UV light, for detecting the presence of said E6 or E7 gene of HPV in the said biological sample;
d. determining the presence of head and neck squamous cell carcinoma in a subject, wherein the presence of fluorescent bands under UV light is determinative of the presence of diagnosing head and neck squamous cell carcinoma.

### In vitro risk stratification methods for HPV positive head and neck cancer

The quantitative determination of exosomal mRNA levels of E6 and E7 genes can provide for means of correlating the stage of cancer with the expression levels. A correlation between the stage of cancer and the mRNA level can provide adequate means of prognosis and treatment.

### EXAMPLES

To gain a better understanding of the invention described herein, the following examples are set forth. It will be understood that these examples are intended to describe illustrative embodiments of the invention and are not intended to limit the scope of the invention in any way.

### Example 1: Collection of biological samples from subjects

Individuals seeking consultation for newly diagnosed cases of oropharyngeal squamous cell carcinoma at Apollo Gleneagles Hospitals, Kolkata were recruited in the study with informed consent. Patient consent forms were provided in three languages English, Hindi and Telugu and were approved by the Institutional Ethical Committee (Ethical permission No. ETH/2019/001).

Tissue sample was collected during biopsy, kept in RNA later (GCC Biotech, India Pvt. Ltd.) and stored overnight at 4°C in the fridge. The next day the sample was moved to -80°C.

6 ml of whole blood was collected and allowed to clot by leaving it at room temperature. This took around 15-30 minutes. The clot was removed by centrifuging at 1000-2000 X g for 10 minutes in a refrigerated centrifuge. The resulting supernatant designated as serum, was collected and stored in -80°C.

7.5 ml whole blood was collected, inverted 5 times to mix and incubated for 30 minutes at room temperature. The tube was centrifuged at 1000-1300 X g for 15 minutes. The plasma sample was dispensed in 2 collection vials and stored at -80°C.

Saliva was collected in a Saliva Exosome Collection and Preservation Kit (Norgen Biotek Corp., Canada, Catalogue No. 65400), mixed by gentle inversion several times to mix the saliva with the dried preservative and stored at -80°C until the further experiment.

Saliva samples were collected from healthy volunteers at Apollo Health City, Hyderabad, India.

Study protocols were explained to all the individuals providing the tissue, saliva, and serum samples. Informed consent was obtained as per the Declaration of Helsinki.

### Example 2: RNA isolation from tissue samples and cDNA conversion

RNA was isolated using TRIzol^{™} reagent. 1 ml of TRIzol^{™} reagent was added to 50 mg of tissue and homogenized using a hand homogenizer and incubated at room temperature for 5 minutes.

Then 0.2 ml of chloroform was added, and the tube was tilted upside down for 2 seconds and incubated for 2-5 minutes. Later, the tube was centrifuged at 12,000 g for 15 minutes at 4°C. The upper aqueous phase was transferred to a new tube carefully, and ice-cold isopropanol was added to it. It was incubated for 10 minutes at room temperature.

The tube was centrifuged at 12,000 g for 15 minutes at 4°C. The supernatant was discarded carefully without disturbing the pellet using a pipette. 1 ml of 75% ethanol was added, briefly the tube vortexed and centrifuged at 7,500 g for 5 minutes at 4°C. The supernatant was discarded carefully with a pipette and the RNA pellet was air-dried for 10 minutes.

The RNA pellet was re-suspended in 50 µl of RNase free water. The RNA was converted to cDNA on the same day of isolation with iScript cDNA Synthesis Kit (Bio-Rad Laboratories, Inc., US) using the manufacturer's protocol. RNA was quantified using µCuvette Spectrophotometer (ThermoFisher Scientific, US). One µg of RNA was used for cDNA conversion. One µg of total RNA from each experimental sample was mixed with 4 µl of 5X iScript Reaction Mix and 1 µl of iScript Reverse Transcriptase and made up to 20 µl with RNase free water.

The components were mixed with a short spin in a centrifuge and kept in a thermal cycler with the following running conditions: 5 min at 25°C (priming), 20 min at 46°C (reverse transcription) and 1 min at 95°C (reverse transcriptase inactivation). The cDNA was stored in -20°C until further analysis.

### Example 3: Total exosome isolation from serum samples

Exosomes were isolated from serum samples using Total Exosome Isolation Reagent (Invitrogen Corporation, US). The 500 µl of the frozen serum sample was thawed on ice and centrifuged at 2000 x g for 30 min at room temperature. The supernatant was transferred to another tube, mixed with 100 µl of Total Exosome Isolation Reagent and vortexed. The tube was incubated at 4°C for 30 minutes and was then centrifuged at 10,000 x g for 10 minutes. The supernatant was aspirated and discarded without disturbing the pellet. The pellet was then resuspended in 200 µl of PBS. The exosomes were stored in -20°C until further processing.

### Example 4: RNA isolation from exosomes and preparation of cDNA

RNA was isolated from the experimental samples using Exiqon miRCURY^{™} exosome isolation kit for cells, urine and CSF (Qiagen Aarhus A/S, Denmark). Two hundred microlitres of the exosome suspension was mixed with 350 µl of lysis solution and vortexed for 15 sec. Two hundred microlitres of 100% ethanol were then added and the mixture was vortexed for 10 sec.

The mixture was then added to a fresh spin column and centrifuged at 14,000 x g for 1 min. The column was then transferred to another collection tube. The column was then washed thrice by the addition of 400 µl of wash solution and centrifugation at 14,000 x g for 1 min. After transferring to a new collection tube, the spin column was centrifuged at 14,000 x g for 2 min for drying before finally transferring onto an elution tube.

The RNA was eluted with 50 µl of elution buffer by centrifugation at 200 x g for 2 min and 14,000 x g for 1 min. The RNA was converted to cDNA on the same day of isolation with iScript cDNA Synthesis Kit (Bio-Rad Laboratories, Inc., US) using the manufacturer's protocol. RNA was quantified using µCuvette Spectrophotometer (ThermoFisher Scientific, US).

As exosomes contain a meagre amount of RNA, 15 µl of total RNA from each experimental sample was mixed with 4 µl of 5X iScript Reaction Mix and 1 µl of iScript Reverse Transcriptase. The components were mixed with a short spin in a centrifuge and kept in a thermal cycler with the following running conditions: 5 min at 25°C (priming), 20 min at 46°C (reverse transcription) and 1 min at 95°C (reverse transcriptase inactivation). The cDNA was stored in -20°C until further analysis.

### Example 5: Primer Design and kit

Based on the sequence analysis, the primers were designed. For designing the primer, an open-source online primer design software, NCBI primer blast software plus was used. Any designing software known to a skilled person can be used. Preferably, the software can be programmed to avoid issues arising out of sequence quality such as avoidance of repetitive elements.

Two primer sets were created, and the molecular markers are selected from the primer set 1 (E6 primers) and primer set 2 (E7 primers).

GAPDH serves as a prognostic marker in many cancer types including head and neck cancer. Therefore, expression of GAPDH provides evidence as to the presence of malignancy.

| **Gene** | **Forward 5'-3'** | **Reverse 3'-5'** |
|---|---|---|
| HPV16 E6 | CAGGAGCGACCCAGAAAGTT (SEQ ID NO: 3) | GCAGTAACTGTTGCTTGCAGT (SEQ ID NO: 4) |
| HPV16 E7 | GATGAAATAGATGGTCCAGC (SEQ ID NO: 5) | GCTTTGTACGCACAACCGAAGC (SEQ ID NO: 6) |
| GAPD H | TCACCATCTTCCAGGAGCGAG A (SEQ ID NO: 7) | GCAGGAGGCATTGCTGATGATC TT (SEQ ID NO: 8) |

A kit was prepared comprising the following primer set:
a. a forward primer comprising the nucleic acid sequence of SEQ ID NO: 3;
b. a reverse primer comprising the nucleic acid sequence of SEQ ID NO: 4;
c. a forward primer comprising the nucleic acid sequence of SEQ ID NO: 5;
d. a reverse primer comprising the nucleic acid sequence of SEQ ID NO: 6.

### Example 6: Semi-quantitative PCR

A semi-quantitative PCR was performed in which the expression level of E6 and E7 genes of HPV16 in salivary exosomes and tumor tissues were studied.

E6/E7 expression was studied in CaSki cell line. CaSki cell line was established from metastasis in the small bowel mesentery. The cells contain an integrated human papillomavirus type 16 genome (HPV-16) at about 600 copies per cell as well as sequences related to HPV-18.

cDNA was 1:5 diluted with RNase free water and one microliter of this diluted product was added into PCR tubes. Semi-quantitative PCR was done using EmeraldAmp^{®} GT PCR Master Mix (TAKARA BIO INC.) using the manufacturer's protocol.

PCR master mix was prepared so that each 9 µl of the master mix contained 5 µl of 2X EmeraldAmp^{®} GT PCR Master Mix, 0.2 µl of 500 nM each forward and reverse primers and 3.8 µl of RNase-free water.

The components of the master mix were shortly spun in a centrifuge and nine µl of the master mix was added to the PCR tubes. No template control (NTC) with 1 µl of DNase-free water instead of cDNA for detecting any DNA contamination.

Semi-quantitative PCR was done in Eppendorf thermocycler with the 3 step temperature cycles.

Initial denaturation at 95°C for 5 mins followed by the three steps of the annealing cycling conditions which were repeated for 35 cycles:
(i) 30 sec at 95°C (denaturation)
(ii) 1 minute at 60°C (annealing temperature of the primer); and
(iii) 1 minute at 72°C (extension) followed by a final extension at 72°C for 5 minutes.

Five µl of the PCR template is run on gel with a 50 Kb ladder to identify the bands against controls. Proper controls (SiHa, Caski) were run along with patient samples.

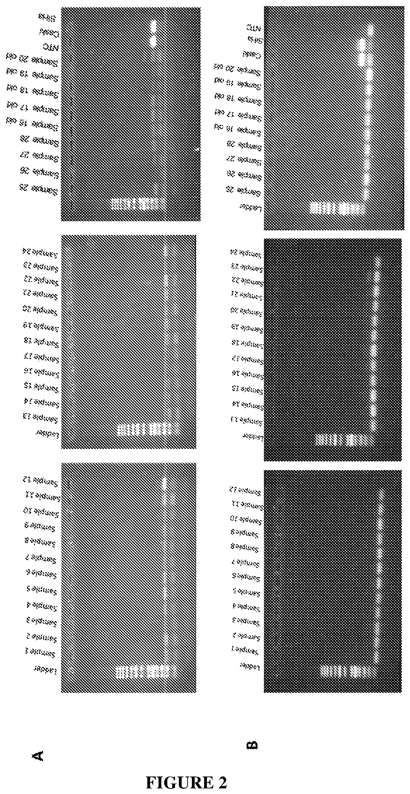

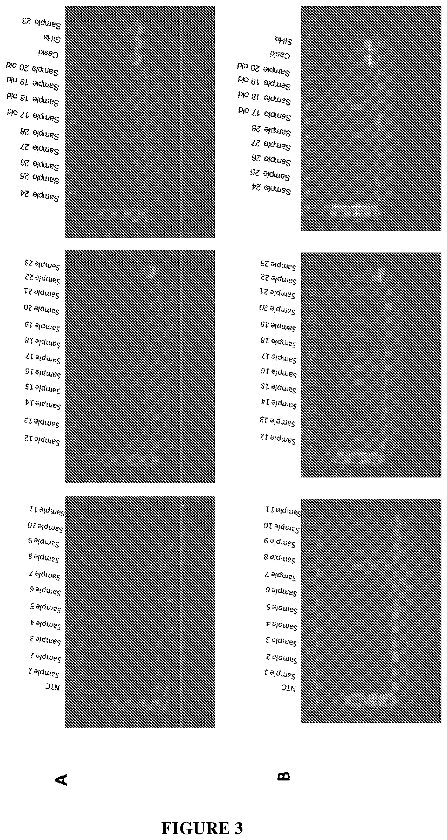

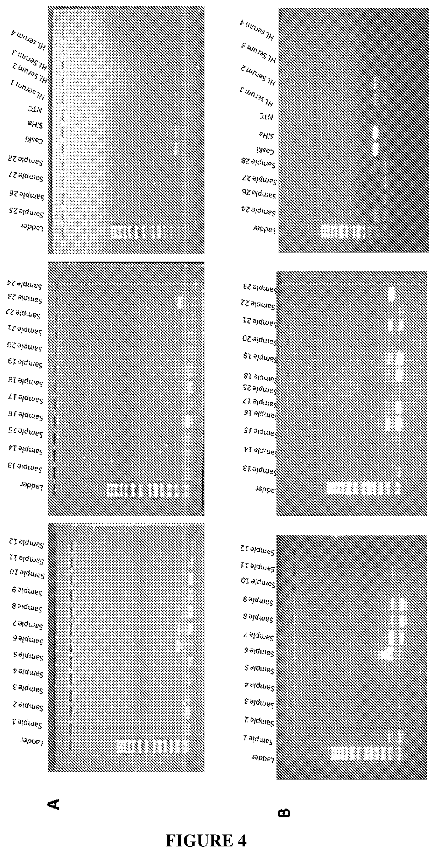

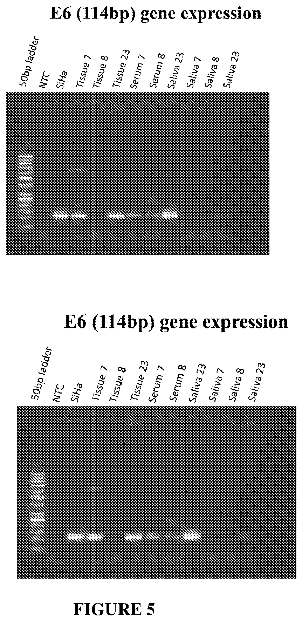

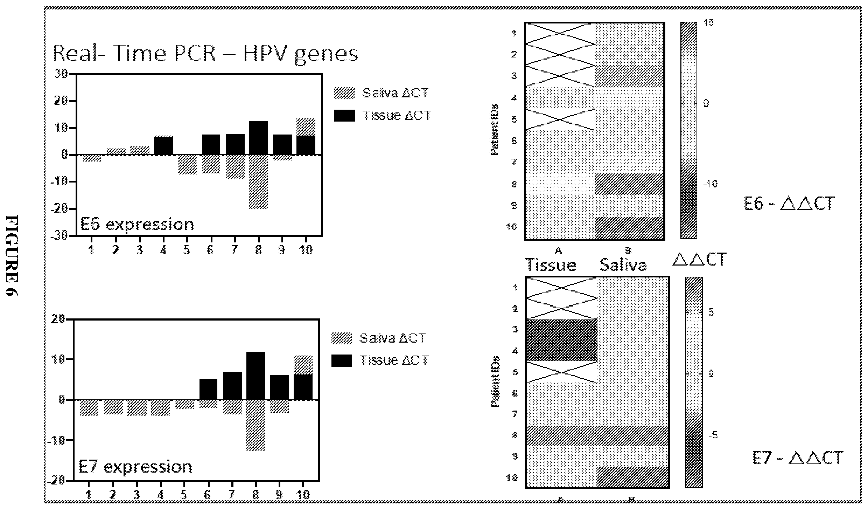

The results of semi quantitative PCR are provided in Figures 2-6.

### Example 7: Quantitative PCR

In order to validate the assay, E6 and E7 genes of HPV16 in exosomes samples were amplified using quantitative PCR.

E6/E7 expression was studied in CaSki cell line. CaSki cell line was established from metastasis in the small bowel mesentery. The cells contain an integrated human papillomavirus type 16 genome (HPV-16) at about 600 copies per cell as well as sequences related to HPV-18.

One micro liter of the 1:5 diluted cDNA was added into the 96-well PCR plate (ThermoFisher Scientific, US). Quantitative PCR was done using iTaq^{™} Universal SYBR ^{®} Green supermix (Bio-Rad Laboratories, Inc., US) using the manufacturer's protocol. PCR master mix was prepared so that so that each 9 µl of the master mix contained 5 µl of 2X iTaq^{™} Universal SYBR ^{®} Green Supermix, 0.2 µl of 500 nM each forward and reverse primers and 3.8 µl of DNase-free water.

The components of the master mix were shortly spun in a centrifuge and 9 µl of the master mix was added to the 96-well PCR plate. No template control (NTC) with 1 µl of DNase-free water instead of cDNA was used for detecting DNA contamination. Quantitative PCR was done in Applied Biosystems^{™} 7500 Real-Time PCR System (ThermoFisher Scientific, US) with the holding stage of 30 sec at 95°C.

The two steps of the cycling conditions were repeated for 40 cycles:
(i) denaturation at 15 sec at 95°C;
(ii) annealing and extension at 60°C for 1 minute (annealing temperature of the primer).

The melting curve stage had the following conditions: 15 sec at 95°C, 1 min at 60°C, 30 sec at 95°C and 15 sec at 60°C.

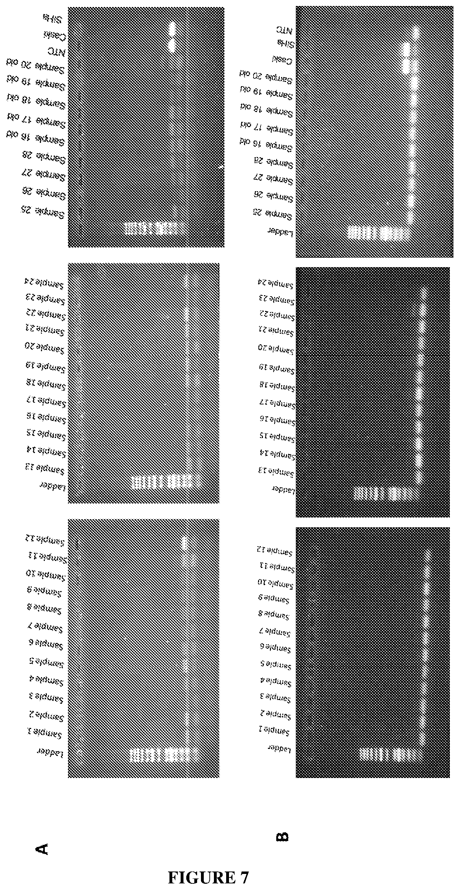

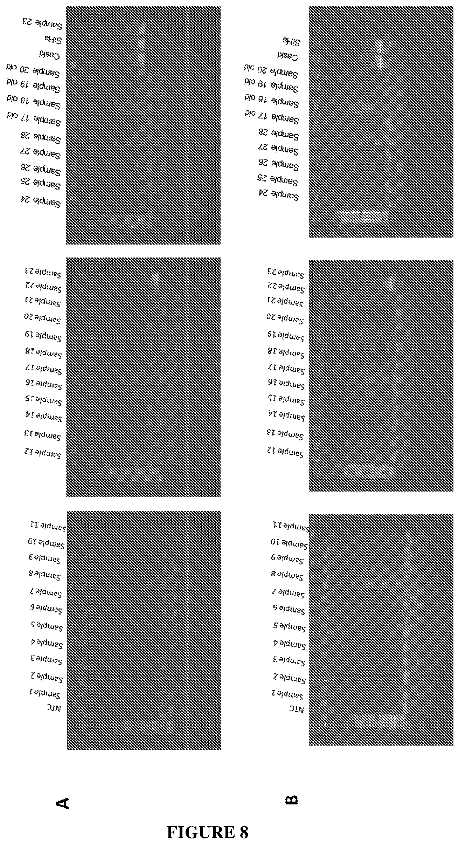

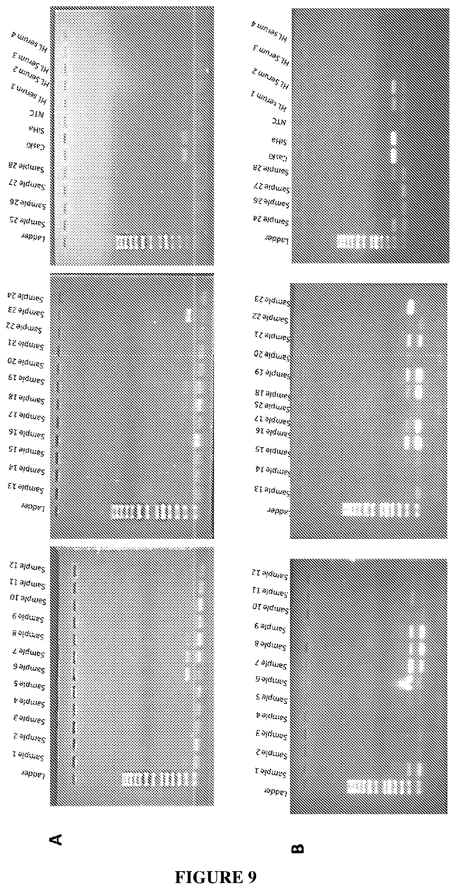

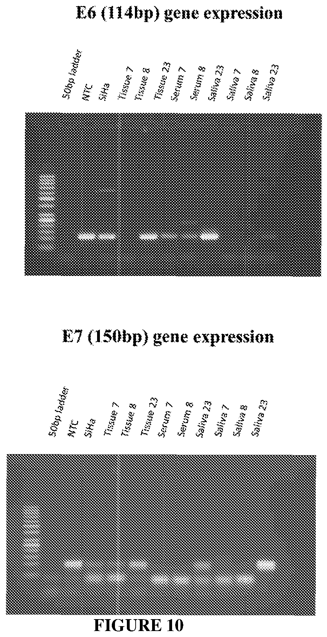

The results of quantitative PCR are provided in Figures 7-10.

### Example 8: Sensitivity and Specificity of the PCR assays

The results were compared with paraffin embedded tissue specimens undergoing HPV In Situ Hybridization (ISH) and p16 Immunohistochemistry (p16 IHC) examination to detect the HPV status. The mean interval between the collection of specimens and histologic diagnosis was 2 months. The results were statistically analysed. The total number of samples collected and analyzed were 33.

The diagnostic accuracy between several sample groups, as provided below were assessed:
- p16 IHC and Tissue E6
- p16 IHC and Tissue E7
- Tissue E6 and Saliva exosome E6
- Tissue E6 and Serum exosome E6
- Tissue E7 and Saliva exosome E7
- Tissue E7 and Serum exosome E7
- Tissue E6 and combination of Saliva + Serum exosome E6
- Tissue E7 and combination of Saliva + Serum exosome E7

The optimal sensitivity of the assays was found to be 66.67% with an optimal 95% Confidence Interval (CI) at 99.16%. The optimal specificity of the assay was found to be 86.67% with an optimal 95% CI at 96.24%. The optimal positive likelihood ratio was 4.0 and optimal negative likelihood ratio was 0.40. The optimal accuracy was found to be 81.82%.

It was noted that exosomal samples containing the combination of exosomes from saliva and serum provides optimal sensitivity and specificity.

## Claims

1. A PCR based method for detecting the presence of E6 or E7 gene of HPV16 in an exosomal sample, said method including the steps of:
a. isolating mRNA from exosomal samples;
b. subjecting the isolated mRNA to amplification by said PCR using primers selected from a group including SEQ ID NO: 3, SEQ ID NO: 4, SEQ ID NO: 5 and SEQ ID NO: 6, wherein said primer sequences are specific to bind to HPV16 gene or fragment thereof; and
c. subjecting the amplified product resulting from step (b) to electrophoresis and visualizing the fluorescent bands under UV light, for detecting the presence of said E6 or E7 gene of HPV in said biological sample.

2. The method as claimed in claim 2, wherein said primer set is adapted to amplify SEQ ID NO: 1 and SEQ ID NO: 2.

3. An in vitro non-invasive method of diagnosing head and neck squamous cell carcinoma in a subject, including:
a. isolating mRNA from exosomal samples of the subject;
b. amplifying, with a pair of PCR primers a target sequence of HPV16 mRNA in the exosomal sample, wherein the target sequence has a nucleic acid sequence of SEQ ID NO: 1 or SEQ ID NO: 2;
c. subjecting the amplified product resulting from step (b) to electrophoresis and visualizing fluorescent bands under UV light, for detecting the presence of said E6 or E7 gene of HPV in said biological sample;
d. determining the presence of head and neck squamous cell carcinoma in a subject, wherein the presence of fluorescent bands under UV light is determinative of the presence of diagnosing head and neck squamous cell carcinoma.

4. The method as claimed in claim 3, wherein the amplification is conducted using a set of amplification primers, wherein the amplification primer set includes:
a. a forward primer comprising the nucleic acid sequence of SEQ ID NO: 3;
b. a reverse primer comprising the nucleic acid sequence of SEQ ID NO: 4;
c. a forward primer comprising the nucleic acid sequence of SEQ ID NO: 5;
d. a reverse primer comprising the nucleic acid sequence of SEQ ID NO: 6.

5. The method as claimed in claim 1 or claim 3, wherein the amplification of the target sequence of HPV16 mRNA in the exosomal sample is performed using semi-quantitative or quantitative PCR.

6. The method as claimed in claim 5, wherein the steps of semi-quantitative PCR included:
a. initial denaturation at 95 °C for 5 minutes;
b. thirty-five annealing cycles, each annealing cycle including:
i. denaturation at 95°C for 30 seconds;
ii. annealing at 60°C for 1 minute;
iii. extension at 72°C for 1 minute; and
iv. a final extension at 72°C for 5 min.

7. The method as claimed in claim 5, wherein the steps of quantitative PCR included:
a. initial denaturation at 95°C for 30 seconds;
b. forty annealing and extension cycles, each cycle comprising:
i. denaturation at 95°C for 15 seconds; and
ii. annealing and extension at 60°C for 1 minute.

8. Use of a primer in a method according to any of claims 1 to 7, wherein the primer is selected from a group including SEQ ID NO: 3, SEQ ID NO: 4, SEQ ID NO: 5 and SEQ ID NO: 6.

9. The use as claimed in claim 8, wherein said primer is adapted to amplify SEQ ID NO:1 or SEQ ID NO: 2.

10. Use of a kit in a method according to any of claims 1 to 7, comprising at least one primer set for amplifying a target sequence of HPV16 exosomal mRNA in a biological sample, wherein the target sequence has a nucleic acid sequence of SEQ ID NO: 1 or SEQ ID NO: 2.

11. The use as claimed in claim 10, wherein the primer set includes:
a. a forward primer comprising the nucleic acid sequence of SEQ ID NO: 3;
b. a reverse primer comprising the nucleic acid sequence of SEQ ID NO: 4;
c. a forward primer comprising the nucleic acid sequence of SEQ ID NO: 5;
d. a reverse primer comprising the nucleic acid sequence of SEQ ID NO: 6.

12. The use as claimed in claim 10, wherein said primer set included:
i. a forward primer comprising the nucleic acid sequence of SEQ ID NO: 3,
ii. a reverse primer comprising the nucleic acid sequence of SEQ ID NO: 4, wherein the primer set is adapted for amplifying a target sequence of E6 gene comprising the nucleic acid sequence of SEQ ID NO: 1.

13. The use as claimed in claim 10, wherein said primer set included:
i. a forward primer comprising the nucleic acid sequence of SEQ ID NO: 5,
ii. a reverse primer comprising the nucleic acid sequence of SEQ ID NO: 6, wherein the primer set is adapted for amplifying a target sequence of E6 gene comprising the nucleic acid sequence of SEQ ID NO: 2.

14. The use as claimed in claim 10, wherein the kit further comprises means to isolate mRNA of HPV16 in a subject, means to carry out amplification of the isolated mRNA, and means to carry out electrophoresing of amplified product and to visualize fluorescent bands under UV light.

## Patentansprüche

1. PCR-basiertes Verfahren zur Detektion der Gegenwart von E6- oder E7-Gen von HPV16 in einer exosomalen Probe, wobei das Verfahren die folgenden Schritte umfasst:
a. Isolieren von mRNA aus exosomalen Proben;
b. Unterziehen der isolierten mRNA einer Amplifizierung durch die PCR unter Verwendung von Primern, die aus einer Gruppe ausgewählt sind, die SEQ.-ID Nr. 3, SEQ.-ID Nr. 4, SEQ.-ID Nr. 5 und SEQ.-ID Nr. 6 einschließt, wobei die Primersequenzen zur Bindung zum HPV16-Gen oder Fragment davon spezifisch sind; und
c. Unterziehen des amplifizierten Produkts, das aus Schritt (b) entsteht, einer Elektrophorese und Visualisieren der Fluoreszenzbanden unter UV-Licht, zur Detektion der Gegenwart des E6- oder E7-Gens von HPV in der biologischen Probe.

2. Verfahren nach Anspruch 2 *[sic],* wobei der Primersatz zur Amplifizierung von SEQ.-**ID** Nr. 1 und SEQ.-ID Nr. 2 geeignet ist.

3. Nicht-invasives in-vitro-Verfahren zur Diagnose von Plattenepithelkarzinomen des Kopfes und Halses bei einem Subjekt, das Folgendes einschließt:
a. Isolieren von mRNA aus exosomalen Proben des Subjekts;
b. Amplifizieren, mit einem Paar von PCR-Primern, einer Zielsequenz von HPV16-mRNA in der exosomalen Probe, wobei die Zielsequenz eine Nukleinsäuresequenz von SEQ.-ID Nr. 1 oder SEQ.-ID Nr. 2 aufweist;
c. Unterziehen des amplifizierten Produkts, das aus Schritt (b) entsteht, einer Elektrophorese und Visualisieren von Fluoreszenzbanden unter UV-Licht, zur Detektion der Gegenwart des E6- oder E7-Gens von HPV in der biologischen Probe;
d. Bestimmen der Gegenwart von Plattenepithelkarzinom des Kopfes und Halses bei einem Subjekt, wobei die Gegenwart von Fluoreszenzbanden unter UV-Licht für die Gegenwart der Diagnose von Plattenepithelkarzinom des Kopfes und Halses bestimmend ist.

4. Verfahren nach Anspruch 3, wobei die Amplifizierung unter Verwendung eines Satzes von Amplifikationsprimern durchgeführt wird, wobei der Amplifikationsprimersatz Folgendes einschließt:
a. einen Vorwärtsprimer, der die Nukleinsäuresequenz von SEQ.-ID Nr. 3 umfasst;
b. einen Rückwärtsprimer, der die Nukleinsäuresequenz von SEQ.-ID Nr. 4 umfasst;
c. einen Vorwärtsprimer, der die Nukleinsäuresequenz von SEQ.-ID Nr. 5 umfasst;
d. einen Rückwärtsprimer, der die Nukleinsäuresequenz von SEQ.-ID Nr. 6 umfasst.

5. Verfahren nach Anspruch 1 oder Anspruch 3, wobei die Amplifizierung der Zielsequenz von HPV16-mRNA in der exosomalen Probe unter Verwendung von halbquantitativer oder quantitativer PCR durchgeführt wird.

6. Verfahren nach Anspruch 5, wobei die Schritte von halbquantitativer PCR Folgendes einschließen:
a. anfängliche Denaturierung bei 95 °C für 5 Minuten;
b. fünfunddreißig Hybridisierungszyklen, wobei jeder Hybridisierungszyklus Folgendes einschließt:
i. Denaturierung bei 95 °C für 30 Sekunden;
ii. Hybridisierung bei 60 °C für 1 Minute;
iii. Elongation bei 72 °C für 1 Minute; und
iv. eine letzte Elongation bei 72 °C für 5 Minuten.

7. Verfahren nach Anspruch 5, wobei die Schritte der quantitativen PCR Folgendes einschließen:
a. anfängliche Denaturierung bei 95 °C für 30 Sekunden;
b. vierzig Hybridisierungs- und Elongationszyklen, wobei jeder Zyklus Folgendes umfasst:
i. Denaturierung bei 95 °C für 15 Sekunden; und
ii. Hybridisierung und Elongation bei 60 °C für 1 Minute.

8. Verwendung eines Primers in einem Verfahren nach einem der Ansprüche 1 bis 7, wobei der Primer aus einer Gruppe ausgewählt ist, die SEQ.-ID Nr. 3, SEQ.-ID Nr. 4, SEQ.-ID Nr. 5 und SEQ.-ID Nr. 6 einschließt.

9. Verwendung nach Anspruch 8, wobei der Primer zur Amplifizierung von SEQ.-ID Nr. 1 oder SEQ.-ID Nr. 2 geeignet ist.

10. Verwendung eines Kits in einem Verfahren nach einem der Ansprüche 1 bis 7, der mindestens einen Primersatz zur Amplifizierung einer Zielsequenz von exosomaler HPV16-mRNA in einer biologischen Probe umfasst, wobei die Zielsequenz eine Nukleinsäuresequenz von SEQ.-ID Nr. 1 oder SEQ.-ID Nr. 2 aufweist.

11. Verwendung nach Anspruch 10, wobei der Primersatz Folgendes einschließt:
a. einen Vorwärtsprimer, der die Nukleinsäuresequenz von SEQ.-ID Nr. 3 umfasst;
b. einen Rückwärtsprimer, der die Nukleinsäuresequenz von SEQ.-ID Nr. 4 umfasst;
c. einen Vorwärtsprimer, der die Nukleinsäuresequenz von SEQ.-ID Nr. 5 umfasst;
d. einen Rückwärtsprimer, der die Nukleinsäuresequenz von SEQ.-ID Nr. 6 umfasst.

12. Verwendung nach Anspruch 10, wobei der Primersatz Folgendes einschließt:
i. einen Vorwärtsprimer, der die Nukleinsäuresequenz von SEQ.-ID Nr. 3 umfasst;
ii. einen Rückwärtsprimer, der die Nukleinsäuresequenz von SEQ.-ID Nr. 4 umfasst, wobei der Primersatz zur Amplifizierung einer Zielsequenz von E6-Gen geeignet ist, die die Nukleinsäuresequenz von SEQ.-ID Nr. 1 umfasst.

13. Verwendung nach Anspruch 10, wobei der Primersatz Folgendes einschließt:
i. einen Vorwärtsprimer, der die Nukleinsäuresequenz von SEQ.-ID Nr. 5 umfasst;
ii. einen Rückwärtsprimer, der die Nukleinsäuresequenz von SEQ.-ID Nr. 6 umfasst, wobei der Primersatz zur Amplifizierung einer Zielsequenz von E6-Gen geeignet ist, die die Nukleinsäuresequenz von SEQ.-ID Nr. 2 umfasst.

14. Verwendung nach Anspruch 10, wobei der Kit weiter Folgendes umfasst: Mittel zur Isolierung von mRNA von HPV16 in einem Subjekt, Mittel zur Durchführung von Amplifizierung der isolierten mRNA und Mittel zur Durchführung von Elektrophorese des amplifizierten Produkts und zur Visualisierung von Fluoreszenzbanden unter UV-Licht.

## Revendications

1. Méthode basée sur la PCR et destinée à la détection de la présence du gène E6 ou E7 du HPV16 dans un échantillon exosomal, ladite méthode comprenant les étapes consistant à :
a. isoler de l'ARNm à partir d'échantillons exosomaux ;
b. soumettre l'ARNm isolé à une amplification par ladite PCR en utilisant des amorces choisies dans le groupe constitué par SEQ ID NO: 3, SEQ ID NO: 4, SEQ ID NO: 5 et SEQ ID NO: 6, où lesdites séquences d'amorces sont spécifiques pour se lier au gène HPV16 ou à un fragment de celui-ci ; et
c. soumettre le produit amplifié résultant de l'étape (b) à une électrophorèse et visualiser les bandes fluorescentes sous lumière UV, afin de détecter la présence dudit gène E6 ou E7 du HPV dans ledit échantillon biologique.

2. Méthode selon la revendication 2 [*sic*]*,* dans laquelle ledit ensemble d'amorces est adapté pour amplifier SEQ ID NO: 1 et SEQ ID NO: 2.

3. Méthode *in vitro* non invasive de diagnostic du carcinome épidermoïde de la tête et du cou chez un sujet, comportant les étapes consistant à :
a. isoler de l'ARNm à partir d'échantillons exosomaux chez le sujet ;
b. amplifier, à l'aide d'une paire d'amorces PCR, une séquence cible de l'ARNm du HPV16 dans l'échantillon exosomal, où la séquence cible a une séquence d'acide nucléique de SEQ ID NO: 1 ou SEQ ID NO: 2 ;
c. soumettre le produit amplifié résultant de l'étape (b) à une électrophorèse et visualiser les bandes fluorescentes sous lumière UV, afin de détecter la présence dudit gène E6 ou E7 du HPV dans ledit échantillon biologique ;
d. déterminer la présence d'un carcinome épidermoïde de la tête et du cou chez un sujet, où la présence de bandes fluorescentes sous lumière UV est un facteur déterminant de la présence diagnostiquée d'un carcinome épidermoïde de la tête et du cou.

4. Méthode selon la revendication 3, dans laquelle l'amplification est réalisée en utilisant un ensemble d'amorces d'amplification, où l'ensemble d'amorces d'amplification comporte :
a. une amorce sens comprenant la séquence d'acide nucléique de SEQ ID NO: 3 ;
b. une amorce antisens comprenant la séquence d'acide nucléique de SEQ ID NO: 4 ;
c. une amorce sens comprenant la séquence d'acide nucléique de SEQ ID NO: 5 ;
d. une amorce antisens comprenant la séquence d'acide nucléique de SEQ ID NO: 6.

5. Méthode selon la revendication 1 ou la revendication 3, dans laquelle l'amplification de la séquence cible de l'ARNm du HPV16 dans l'échantillon exosomal est réalisée à l'aide d'une PCR semi-quantitative ou quantitative.

6. Méthode selon la revendication 5, dans laquelle les étapes de PCR semi-quantitative comportent :
a. une dénaturation initiale à 95°C pendant 5 minutes ;
b. trente-cinq cycles d'hybridation, chaque cycle d'hybridation comportant :
i. une dénaturation à 95°C pendant 30 secondes ;
ii. une hybridation à 60°C pendant 1 minute ;
iii. une élongation à 72°C pendant 1 minute ; et
iv. une élongation finale à 72°C pendant 5 minutes.

7. Méthode selon la revendication 5, dans laquelle les étapes de PCR quantitative comportent :
a. une dénaturation initiale à 95°C pendant 30 secondes ;
b. quarante cycles d'hybridation et d'élongation, chaque cycle comprenant :
i. une dénaturation à 95°C pendant 15 secondes ; et
ii. une hybridation et une élongation à 60°C pendant 1 minute.

8. Utilisation d'une amorce dans une méthode selon l'une quelconque des revendications 1 à 7, dans laquelle l'amorce est choisie dans le groupe constitué par SEQ ID NO: 3, SEQ ID NO: 4, SEQ ID NO: 5 et SEQ ID NO: 6.

9. Utilisation selon la revendication 8, dans laquelle ladite amorce est adaptée pour amplifier SEQ ID NO: 1 ou SEQ ID NO: 2.

10. Utilisation d'un kit dans un méthode selon l'une quelconque des revendications 1 à 7, comprenant au moins un ensemble d'amorces pour amplifier une séquence cible d'ARNm exosomal du HPV16 dans un échantillon biologique, où la séquence cible a une séquence d'acide nucléique de SEQ ID NO: 1 ou SEQ ID NO: 2.

11. Utilisation selon la revendication 10, dans laquelle l'ensemble d'amorces comporte :
a. une amorce sens comprenant la séquence d'acide nucléique de SEQ ID NO: 3 ;
b. une amorce antisens comprenant la séquence d'acide nucléique de SEQ ID NO: 4 ;
c. une amorce sens comprenant la séquence d'acide nucléique de SEQ ID NO: 5 ;
d. une amorce antisens comprenant la séquence d'acide nucléique de SEQ ID NO: 6.

12. Utilisation selon la revendication 10, dans laquelle ledit ensemble d'amorces comporte :
i. une amorce sens comprenant la séquence d'acide nucléique de SEQ ID NO: 3,
ii. une amorce antisens comprenant la séquence d'acide nucléique de SEQ ID NO: 4, où l'ensemble d'amorces est adapté pour amplifier une séquence cible du gène E6 comprenant la séquence d'acide nucléique de SEQ ID NO: 1.

13. Utilisation selon la revendication 10, dans laquelle ledit ensemble d'amorces comporte :
i. une amorce sens comprenant la séquence d'acide nucléique de SEQ ID NO: 5,
ii. une amorce antisens comprenant la séquence d'acide nucléique de SEQ ID NO: 6, où l'ensemble d'amorces est adapté pour amplifier une séquence cible du gène E6 comprenant la séquence d'acide nucléique de SEQ ID NO: 2.

14. Utilisation selon la revendication 10, dans laquelle le kit comprend en outre un moyen pour isoler l'ARNm du HPV16 chez un sujet, un moyen pour réaliser l'amplification de l'ARNm isolé, et un moyen pour réaliser l'électrophorèse du produit amplifié et pour visualiser les bandes fluorescentes sous lumière UV.
